(19) **Europäisches Patentamt / European Patent Office / Office européen des brevets**

(11) **EP 4 119 049 A1**

(12) **EUROPEAN PATENT APPLICATION**

(43) Date of publication:
**18.01.2023 Bulletin 2023/03**

(21) Application number: **21184993.0**

(22) Date of filing: **12.07.2021**

(51) International Patent Classification (IPC):
**A61B 5/11** (2006.01)    **A61B 5/00** (2006.01)
**A63B 69/00** (2006.01)

(52) Cooperative Patent Classification (CPC):
**A61B 5/1118; A61B 5/112; A61B 5/1123;
A61B 5/7246; A61B 5/7257;** A61B 5/1112;
A61B 5/742

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Polar Electro Oy
90440 Kempele (FI)**

(72) Inventor: **Majava, Ville
90440 Kempele (FI)**

(74) Representative: **Kolster Oy Ab
Salmisaarenaukio 1
P.O. Box 204
00181 Helsinki (FI)**

(54) **METHOD FOR MEASURING EFFECTIVENESS OF PERIODIC MOTION**

(57) The present document discloses a solution for estimating effectiveness of periodic motion during a physical exercise such as a running exercise. According to an aspect, a computer-implemented method for estimating the effectiveness of the periodic motion comprises: measuring, by using at least one motion sensor, periodic motion of a user and thus acquiring motion measurement data during a time interval of a physical exercise; transforming the motion measurement data into frequency-domain samples; extracting, amongst the frequency-domain samples by using peak detection, a first subset of frequency-domain samples representing periodic motion; computing a metric indicating a ratio between energy on the first subset of frequency domain samples and energy on other frequency domain samples; and mapping the computed ratio to an effectiveness parameter by using a determined mapping rule and outputting the effectiveness parameter via an interface.

Fig 2

## Description

### TECHNICAL FIELD

The present invention relates to a field of motion measurements in the context of physical exercises and, in particular, to signal processing for determining effectiveness of periodic motion.

### TECHNICAL BACKGROUND

Running effectiveness can be measured by using motion sensors and estimating various parameters on the basis of the measurements. Similarly, the effectiveness of other sports such as swimming or walking can be determined. Generally, the motion can be considered efficient when it directs the person ahead and, on the other hand, strong vertical or lateral motion trajectories indicate inefficient motion. Further improvements are, however, needed.

### BRIEF DESCRIPTION

The present invention is defined by the subject matter of the independent claims.
Embodiments are defined in the dependent claims.

### BRIEF DESCRIPTION OF THE DRAWINGS

In the following the invention will be described in greater detail by means of preferred embodiments with reference to the accompanying drawings, in which

> Figure 1 illustrates a measurement arrangement to which embodiments of the invention may be applied;
> Figure 2 illustrates a flow diagram of an embodiment of a process for estimating effectiveness of periodic motion conducted during a physical exercise;
> Figures 3A, 3B, and 3C illustrate frequency domain representations of exemplary motion measurement data;
> Figure 4 illustrates presentation of effectiveness according to an embodiment;
> Figure 5 illustrates a flow diagram of a process for correcting a running power parameter;
> Figure 6 illustrates a flow diagram of a process for monitoring running effectiveness according to an embodiment;
> Figure 7 illustrates a procedure for improving accuracy of motion effectiveness estimation according to an embodiment; and
> Figure 8 illustrates an embodiment of an apparatus or a system for estimating effectiveness of motion.

### DETAILED DESCRIPTION OF THE INVENTION

The following embodiments are exemplifying.

Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

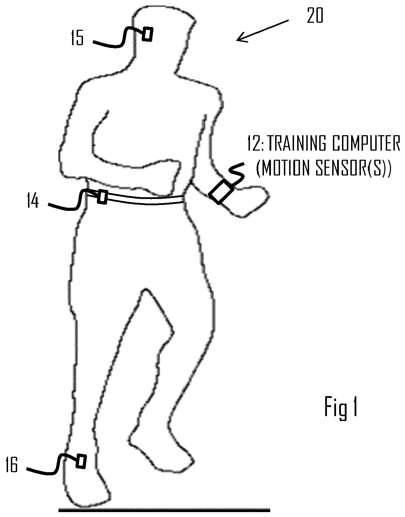

Figure 1 illustrates a measurement system comprising sensor devices that may be used in the context of some embodiments of the present invention. The sensors may employ one or more measurement technologies for measuring motion of a user 20 during a physical exercise where the user performs periodic motion, such as running, walking, or swimming. At least one motion sensor device 12, 14, 15, 16 may be attached to the user while the user is performing the exercise. The motion sensor may be attached to any body part of the user 20, e.g. to a hand, torso, head, or foot, as illustrated in Figure 1. The motion sensors may comprise one or more accelerometers configured to measure acceleration of the user's body part(s) in one, two or three spatial dimensions. The motion sensors may comprise a gyroscope configured to measure rotation of the user's body part(s). Magnetometer is yet another embodiment of the motion sensor. A magnetometer is configured to measure the motion by measuring changes in magnetic fields. Some embodiments employ a sensor fusion comprising a plurality of motion sensors of different types, even a fusion of a three-dimensional accelerometer, a gyroscope, and a magnetometer. At least some of the motion sensors may be comprised in a wearable training computer such as a wrist computer.

During a physical exercise such as running, the user works to move his/her body forward. However, due to the inefficient technique, terrain, and obstacles, the work may be diverted to other directions as well. As a consequence, the work (or equivalently mechanical energy) the user performs during the periodic motion can be expressed as:

$$W_{tot} = W_{run} + W_{waste}$$

where Wtot is the total work consisting of the work $W_{run}$ that directs the user forward and the work $W_{waste}$ that is diverted to other directions and absorbed by the terrain. Effectiveness (equivalent to efficiency) M of the motion may then be represented as:

$$M = W_{run} / W_{tot}$$

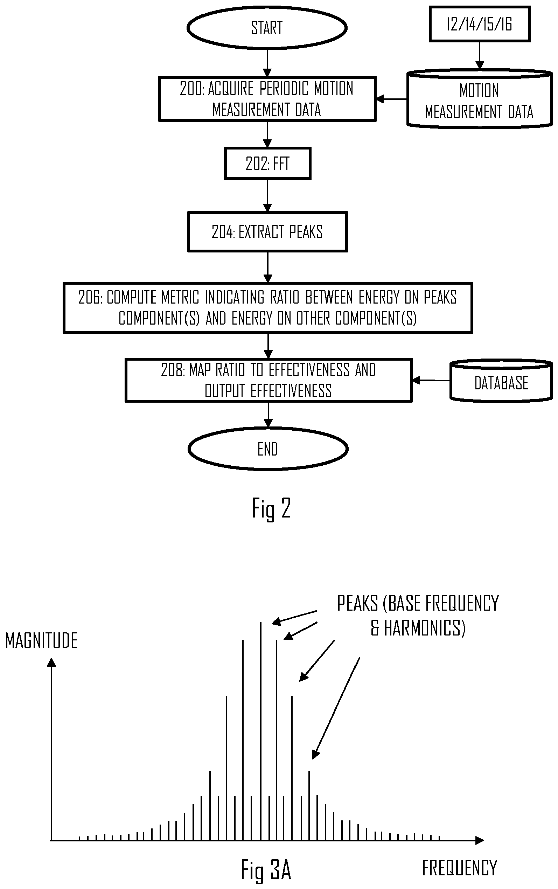

As described above, at least one of the above-described motion sensors may be configured to measure the user's periodic motion during the physical exercise. Thus acquired motion measurement data may have been measured during a time interval that forms a part of the physical exercise, or the motion measurement data may comprise all motion measurement data acquired during the exercise. The motion measurement data may be used in a computer-implemented method for estimating effectiveness of the periodic motion. Figure 2 illustrates a flow diagram of such a method, comprising: transforming the motion measurement data (acquired in block 200) into frequency-domain samples, e.g. by using a fast Fourier transform (FFT) in block 202; extracting (block 204), amongst the frequency-domain samples by using peak detection, a first subset of frequency-domain samples representing periodic motion; computing (block 206) a metric indicating a ratio between energy on the first subset of frequency domain samples and energy on other frequency domain samples; and mapping (block 208) the computed ratio to an effectiveness parameter by using a determined mapping rule and outputting the effectiveness parameter via an interface. The interface may be a user interface, a communication interface, or a memory interface.

Now, the energy on the first subset of samples may be directly proportional to $W_{run}$, and Wtot may be the total amount of energy on the frequency-domain samples. As a consequence, the metric may directly represent M or at least be proportional to the effectiveness M as 'aM+b' where 'a' and 'b' are a scaling factor and an offset factor that may be selected according to the implementation. Accordingly, the effectiveness can be determined with low computational complexity. The mapping may map the metric to a determined scale.

Since the periodic motion results in motion measurement data having periodic signal components, the components representing the periodic motion are detectable in the frequency domain samples as peaks and can thus be extracted by using conventional peak detection algorithms known in the art.

In the embodiment of Figure 2, the ratio between the energy on the periodic signal components with respect to the energy on the other signal components or the total energy is the indicator of the effectiveness of the motion, e.g. running or swimming. The more energy is on the periodic components, the smoother the running is, and less work is used to change the user's 20 motion vector, thus providing a greater effectiveness. From that perspective, the mapping rule may map a ratio indicating more energy in the first subset of frequency-domain samples (periodic part) to a greater effectiveness parameter.

The frequency-domain representation may include a signal component representing the base frequency (centre frequency) and harmonic signal components representing harmonic distortion between signal components on the base frequency and at least one different frequency. The harmonic signal components may then be understood as signal components that are also beneficial to the effectiveness and are thus included in the first subset. In an embodiment, the metric is the energy on the harmonic signal components with respect to the total energy or the energy on the signal components other than the harmonic signal components. In such a case, even the base frequency may be omitted from the first subset. In another embodiment, the frequency-domain sample representing the base frequency is also included in the first subset. In such a case, the metric is then proportional to a signal-to-noise ratio where the signal is represented by the first subset while the noise is represented by the other frequency-domain samples excluding the first subset.

In an embodiment, the metric is proportional to a total harmonic distortion known in the art of signal processing. The total harmonic distortion may in this case be computed as the amount of energy on the harmonic signal components with respect to the amount of energy on the base (centre) frequency. The first subset thus includes the harmonic signal components without the base frequency. The effectiveness parameter may then be proportional to the total harmonic distortion. While the base frequency may be assumed to represent the periodic motion that would bring the user forward, it has been surprisingly found out that the total harmonic distortion (the amount of energy on the harmonic components with respect to the energy on the base frequency) also correlates with the effectiveness of the motion. When the motion is inefficient, the total harmonic distortion is lower than when the motion is efficient.

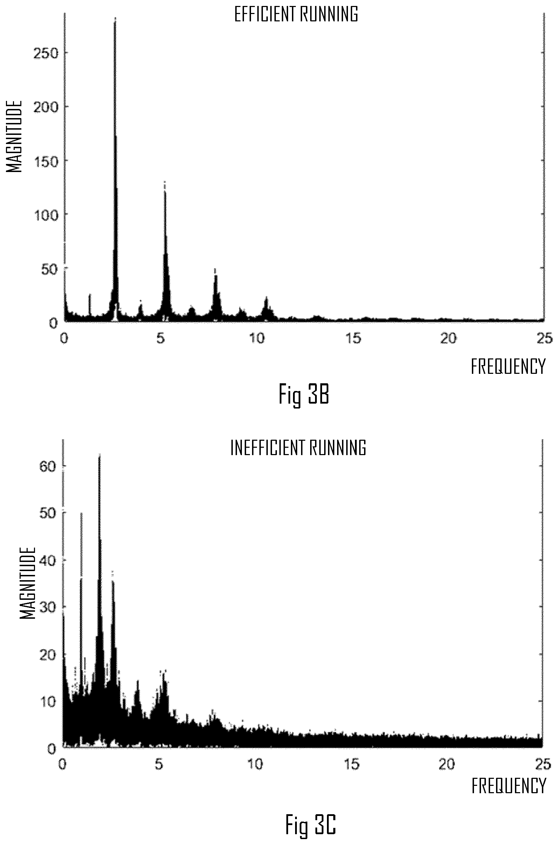

Figures 3A to 3C illustrate example distributions of the frequency-domain samples. Since the motion is periodic, there exist signal components that distinguish from other signal components and that can be detected as peaks. The other signal components represent noise and motion components that direct the user to other directions than forward. Depending on the amount of measurement data samples and the applied signal processing, there may be one or several peaks in the frequency-domain representation of the motion measurement data: one peak at the base (central) frequency and its harmonics. Typically, there is a substantial amount of energy on the other signal components that exist because of the non-ideal conditions such as terrain, weather, the user's non-ideal stepping, etc. Figures 3B and 3C illustrate frequency-domain representations of measurement data measured during efficient running (Figure 3B) and inefficient running (Figure 3C). In this situation the inefficiency is caused by the terrain. In the case of Figure 3B, the terrain is flat while the measurement data of Figure 3C has been acquired when running on a forest trail where the user has to side-step to keep the balance. It can be observed that there is less noise components and higher peaks when the running is efficient. Accordingly, the amount of energy on the peaks is greater than on the other signal components. Furthermore, it can be observed that the raised noise level may consume harmonic signal components in the measurement data representing the inefficient running, thus reducing the total harmonic distortion. Since more harmonic signal components can be detected in the measurement data representing the efficient running, the total harmonic distortion is greater.

In yet another embodiment, the effectiveness is measured by observing the spectral pattern over multiple sets of time intervals of the exercise. A set of measurement data may be collected during each time interval, and the frequency-domain representation may be computed to each set of measurement data. Then, spectral analysis may be performed by correlating the sets of measurement data. If the correlation is high, high effectiveness may be determined and a greater effectiveness parameter may be output. On the other hand, if the correlation is low, a lower effectiveness parameter may be output. The correlation may comprise correlating the height of the peaks and/or a position of the peaks in the frequency domain. In other words, greater spectral stability over multiple time intervals of the physical exercise may be associated with a greater effectiveness.

In an embodiment, the peak detection is based on setting a threshold for determining the first and second subset of the frequency-domain samples. Those samples that exceed the threshold may be considered as the periodic components and added to the first subset, while the other samples are considered as the aperiodic components or noise and excluded from the first subset. In another embodiment, a window is used to separate the first subset. The window may be set as centred around the base frequency (the highest peak) by using the threshold such that the window expands to both higher and lower frequencies from the centre frequency until a detected peak no longer exceeds the threshold. As a consequence, the window may include also samples that do not themselves count as peaks but are still considered as the periodic components (see samples between the central peaks in Figure 3).

In an embodiment, the time interval spans over the whole physical exercise. In such an embodiment, the effectiveness is computed at the end of the exercise, and all motion measurement data acquired during the exercise are used to compute the effectiveness of the exercise. The effectiveness may then be stored as linked to the exercise in a database storing the user's exercises. The effectiveness may also be output to the user via a user interface such as a display unit of the wrist computer or another personal electronic device, e.g. a smart phone, a tablet computer, or another computer. The database may be stored in the wrist computer, in the personal electronic device, and/or a server computer storing the user's 20 user account.

In another embodiment, the method of Figure 2 is performed by a wearable training computer (e.g. the wrist computer) repeatedly over multiple time intervals during the physical exercise to acquire multiple effectiveness parameters for the physical exercise, and wherein the effectiveness parameters are output via a display unit of the wearable training computer, e.g. during the exercise. The time interval may be long enough to enable sufficient statistics for the computation. Let us remind that the periodicity is typically in the order of 1-3 Hz, and it may take several seconds, e.g. 20 seconds or a minute, to acquire the sufficient statistics. The wearable training computer may perform pre-analysis for selecting the motion measurement data for the computation of the effectiveness. The pre-analysis may include evaluation of the periodicity of the motion measurement data. The pre-analysis may include monitoring the cadence that is conventionally measured in real time as a part of a running exercise, for example. When the cadence is detected to have stayed within a determined range for the determined time interval, the training computer may trigger the computation of the effectiveness for the motion measurement data acquired during the time interval.

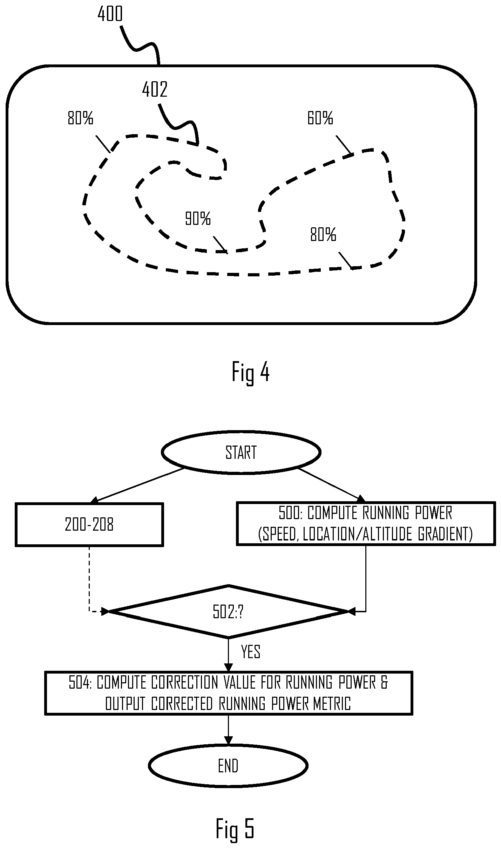

In an embodiment, the method where the effectiveness is computed multiple times during the exercise, the method further comprises outputting, after an end of the exercise, a display view indicating the computed effectiveness parameters along a route of the physical exercise. As known in the art, the exercise may comprise utilization of a global navigation satellite (GNSS, GPS, GLONASS), receiver to position the user 20 during the exercise and to determine the route of the exercise on a geographical map. Figure 4 illustrates an example of such a display view 400 and the route 402 where the effectiveness is illustrated at various locations along the route. When triggering the start of the process of Figure 2, a processor of the training computer may acquire, on the basis of positioning coordinates acquired by using the GNSS receiver, the location(s) of the wearable training computer and the user 20 at the time of acquiring the motion measurement data and store the location(s) together with the computed effectiveness. In the illustration of Figure 4, the effectiveness is illustrated as a percentage value, but any other metric or parameter describing the effectiveness is equally possible. In an embodiment, the effectiveness is illustrated by colour coding. The curve 402 representing the route may be coloured by different colours at different locations of the route, and the colour may represent the effectiveness along the route. A more vivid colour may represent a high effectiveness (e.g. high energy on aperiodic frequency components) while a light or less intense colour may represent low effectiveness.

In an embodiment where the effectiveness parameter is a running effectiveness parameter, the effectiveness parameter is used as a correction factor for a measured running power parameter. Furthermore, a mechanical load of the exercise may be computed on the basis of the corrected running power parameter. Figure 5 illustrates such an embodiment.

Conventionally, the running power may be computed by measuring the user's speed during the exercise and, a gradient in position and/or altitude. The gradient of the position may be acquired by using the GNSS receiver, and the gradient of the altitude may be acquired by using the GNSS receiver and/or an altimeter (a barometer). The position and the altitude may be used to determine whether the user is ascending or descending or running on flat ground, thus scaling the running power on the basis of the terrain slope. The basis for the running power is in the estimation of a mechanical energy produced by the user. The mechanical energy is a combination of kinetic energy (proportional to the speed) and changes in gravitational potential energy (proportional to elevation/altitude). However, the running power computed in such manner may not always be accurate. The running power is thus proportional to the work the user does during the exercise. Referring to Figure 5, the running power is computed in a conventional manner in block 500 for the time interval of the physical exercise. Similarly, blocks 200 to 208 are performed for the time interval of the physical exercise. In block 502, it is determined whether or not to perform the correction of the running power on the basis of the effectiveness parameter. The decision may be based on evaluating the effectiveness parameter. For example, if the effectiveness is below a threshold, it may be determined that the user has performed the exercise in a non-flat terrain where the motion measurement data is substantially aperiodic. In such a case, the correction may be omitted. On the other hand, if the effectiveness is above the threshold, the correction of the running power may be triggered. In an embodiment, the corrected running power is computed in block 504 on the basis of the following Equation:

$$E_{tot} = E_{ext} + E_{int}$$

$$M = \frac{E_{ext}}{E_{tot}}$$

where M is again the metric describing the effectiveness computed according to any one of the above-described embodiments, $E_{ext}$ is the running power multiplied by the duration of the time interval or a corresponding metric computed from the running power and representing the work the user has performed during the time interval. $E_{tot}$ represents total (mechanical) energy produced by the user that can be acquired by measuring the user's heart rate or stroke volume by using conventional means. The heart rate may be computed by measuring an electrocardiogram or a photoplethysmogram of the user 20, while the stroke volume conventionally requires measuring the user's bioimpedance. $E_{int}$ represents a part of the mechanical energy that is spent on inefficient technique or environmental conditions.

The corrected running power may then be acquired by dividing Etot by the duration of the time interval. The corrected running power may then be output via the user interface, for example. The corrected running power may be used to provide more accurate results to other parameters that are conventionally computed on the basis of the running power. Such other parameters include a muscle load of the exercise, which is one indicator of a training load of the exercise, and a recovery estimate indicating a time needed to recover from the exercise.

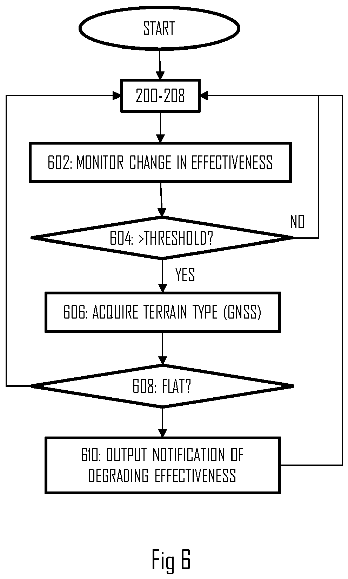

Let us then describe some embodiments where the periodic motion is running and the effectiveness parameter is a running effectiveness parameter. The method may further comprise monitoring for changes in the running effectiveness parameter and, upon detecting a degradation in the running effectiveness parameter, triggering an action to check the basis for the degradation. Figure 6 illustrates such an embodiment of the method. In this embodiment, the running effectiveness is computed repeatedly during the exercise. Referring to Figure 6, after performing block 208, block 602 may be performed to compare the current effectiveness with one or more previous effectiveness values. Upon determining that the change is more than a determined threshold in block 604, the process may proceed to block 606. Otherwise, the process may return to block 200 to acquire the next set of motion measurement data. In block 606, a processing system carrying out the method acquires data indicating a terrain type associated with the measured periodic motion and determines the terrain type from the data. The data may comprise the GNSS data that is mapped to a geographical terrain map to determine the terrain type where the user is currently located. In another embodiment, the terrain type is determined on the basis of altitude measurements. If the terrain type is determined to be non-flat in block 608, the processing system may ignore the change in the running effectiveness parameter and return to block 200. In other words, the processing system may determine that the user cannot affect the change in the running effectiveness but that the change is due to the non-flat terrain. The terrain may be determined to be non-flat directly from the geographical map upon receiving the user's location on the map. From the altitude, the non-flat terrain maybe detected if the altitude is deemed to have changed along with the running effectiveness. On the other hand, if the terrain type is determined to be flat in block 608, the processing system may output an indication to the user of the degradation in the running effectiveness (block 610). The notification may instruct the user to correct the running technique, for example by reducing the speed.

In an embodiment, the accuracy of the effectiveness parameter is improved by determining the direction of motion by using the motion sensors capable of measuring the motion in the X, Y, and Z directions. Upon receiving the motion measurement data from the various motion sensors, the processing system may first align the motion measurement data into the same X, Y, Z coordinate system according to state-of-the-art calibration. Thereafter, the X, Y, and Z components acquired from the various motion sensors may be combined, or X, Y, Z components provided each motion sensor may be processed separately. When computing the effectiveness, each the combined or separate X, Y, Z components of the motion measurement signals may be used in the process of Figure 2. In an embodiment, the effectiveness is computed per X, Y, Z component and, optionally, per motion sensor. Thereafter, the multiple effectiveness values may be aggregated into a single effectiveness parameter in an embodiment of the process of Figure 2.

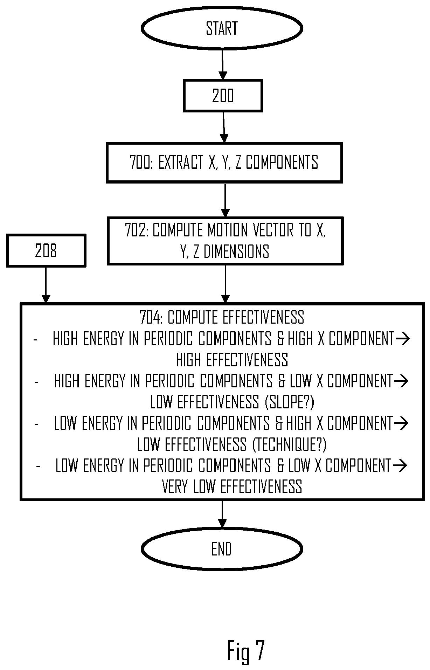

The effectiveness parameter determined in block 208 may then be used as one indicator of the effectiveness of the motion the user is performing. Figure 7 illustrates a process for using the motion measurement data to further improve the accuracy of the effectiveness computation, by using the three-dimensional motion measurement data. As described above, the X dimension represents motion towards a general direction the user is moving, Y dimension represents lateral motion of the user towards a direction that is perpendicular to the X dimension, and Z dimension represents motion to a direction that is perpendicular to both X and Y directions. As described above, the processing system may extract the X, Y, and Z components from the motion measurement data in block 700. In block 702, the processing system computes, on the basis of the motion measurement data, a motion vector for each spatial dimension X, Y, Z and determines the effectiveness in block 704 on the basis of the effectiveness parameter acquired in block 208 and, further, on the basis of the motion vectors such that the running effectiveness is directly proportional to the magnitude of the vector in X dimension and inversely proportional to the magnitude of the vectors in the Y and Z dimensions. Block 704 of Figure 7 illustrates some principles for the correlation between the magnitudes of the vectors, the effectiveness parameter computed in block 208, and the overall effectiveness of the motion the user is performing. In the following, the principles are described in the relative context of high/low amount of energy in the periodic signal components of the frequency-domain representation of the motion measurement data and high/low value of the motion vector in a particular dimension. With respect to the effectiveness parameter computed in block 208, 'high' may refer 'above a determined threshold' to qualify for high effectiveness, while 'low' may refer 'below the determined threshold or another threshold lower than the determined threshold' to qualify for low effectiveness. With respect to the motion vectors, 'high' in a particular dimension may mean a magnitude that is higher than magnitudes in the other two dimensions, while 'low' in the particular dimension may mean a magnitude that is lower than a magnitude of at least one of the other two dimensions. Other interpretations are equally possible. The X components may be of particular relevance in the determination of the effectiveness, and it may be compared with the other dimensions Y and Z. As a consequence, in order for the motion vector X to qualify for 'high', the magnitude in the X vector may need to be substantially greater than magnitudes of the Y and Z components, e.g. by a determined amount. The amount may be determined in a suitable manner, e.g. the X dimension shall carry at least 80, 90, or 95 per cent of the total magnitude. Another threshold may be equally possible.

Let us then elaborate the principles. In case there is high energy on the periodic signal components and the magnitude of the X dimension is sufficiently high, the processing system may output an effectiveness value indicating high effectiveness, e.g. above 75%. In case there is high energy on the periodic signal components but the magnitude of the X dimension is not as high as in the previous case, the processing system may output an effectiveness value indicating lower effectiveness, e.g. between 50-75%, depending on the actual values of the effectiveness parameter received from block 208 and the magnitude of the X dimension. This range may indicate that the low effectiveness may be due to the terrain where the user 20 is capable of maintaining the periodicity in the motion but struggles in focusing the work to the X dimension. In other words, work is also done to move the user in the lateral dimension Y or in up-down motion in the Z dimension. In case there is low energy on the periodic signal components but the magnitude of the X dimension is sufficiently high, the processing system may output an effectiveness value indicating low effectiveness, e.g. between 30-70%, depending on the actual values of the effectiveness parameter received from block 208 and the magnitude of the X dimension. This combination of the effectiveness parameters may indicate that the low effectiveness may be due to poor running technique, where the user is incapable of maintaining the periodicity but is capable of directing the motion towards the X direction. In case there is low energy on the periodic signal components and the magnitude of the X dimension is also low, the processing system may output an effectiveness value indicating very low effectiveness, e.g. between 0-30%, depending on the actual values of the effectiveness parameter received from block 208 and the magnitude of the X dimension. This combination of the effectiveness parameters indicates that the motion is aperiodic and the user is not able to direct the motion appropriately to the X dimension but the direction of the work is too much to the Y and Z dimensions.

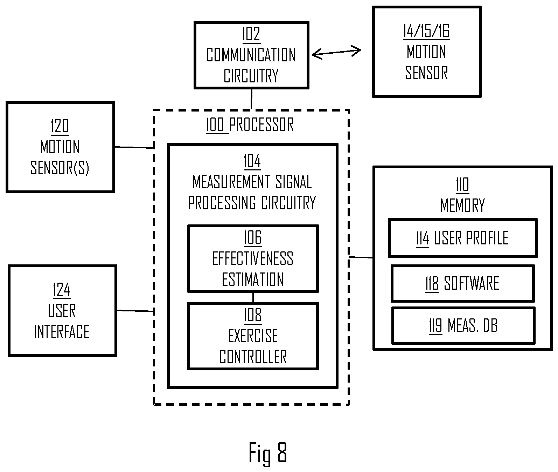

Figure 8 illustrates an embodiment of an apparatus or apparatuses (a system) configured to carry out at least some of the above-described functions in estimating the effectiveness of the motion the user is performing during the exercise. The apparatus may comprise an electronic device comprising the above-described processing system configured to carry out the method of Figure 2 or any one of the embodiments thereof. The apparatus may be the wearable training computer, the personal electronic device, or the server computer. The processing system may comprise at least one processor 100 and at least one memory 110. The apparatus may further comprise a user interface 124 comprising a display screen or another display unit, an input device such as one or more buttons and/or a touch-sensitive surface, and an audio output device such as a loudspeaker. In some embodiments, the user interface 124 comprises a haptic output device configured to provide haptic indications to the user 20.

The processor 100 may comprise a measurement signal processing circuitry 104 configured to estimate the effectiveness by carrying out the procedure of Figure 2. The measurement signal processing circuitry 104 may comprise an effectiveness estimation circuitry 106 configured to estimate the motion effectiveness according to any one of the above-described embodiments. The effectiveness estimation circuitry 106 may output the computed effectiveness to the user interface 124, to a communication circuitry 102, or to another signal processing circuitry of the apparatus estimating another parameter using the motion effectiveness as an input, e.g. the muscle load or the corrected running power.

The apparatus may comprise the communication circuitry 102 connected to the processor 100. The communication circuitry may comprise hardware and software suitable for supporting Bluetooth® communication protocol such as Bluetooth Smart specifications. It should be appreciated that other communication protocols are equivalent solutions as long as they are suitable for establishing a personal area network (PAN) or suitable for measurement scenarios described in this document. The processor 100 may use the communication circuitry 102 to transmit and receive frames according to the supported wireless communication protocol. The frames may carry a payload data comprising the above-described motion measurement data measured by one or more external motion sensors 14, 16, such as the motion measurement data required for the estimation of the effectiveness. In some embodiments, the processor 100 may use the communication circuitry 102 to transmit the motion measurement data, estimated effectiveness, and/or other parameters to another apparatus, e.g. to a cloud server storing the user's 20 user account or to the personal electronic device.

In an embodiment, the apparatus comprises at least one motion sensor 120, e.g. any one or more of the above-described motion sensors. Additionally, the apparatus may communicate with at least one external motion sensor 14, 16 through the communication circuitry 102.

In embodiments where the motion sensors are provided in different, physically separate devices, the devices may be synchronized to a common clock such as a clock of Global Positioning System or another satellite navigation system providing an accurate clock signal for both devices. Some wireless communication protocols provide synchronization tools, and some embodiments may use such tools to carry out the synchronization. One of the devices may operate as a master clock and it may transmit a frame indicating its clock value to the other device(s), thereby providing clock synchronization.

The processor 100 may further comprise an exercise controller 108 configured to control the motion sensors 120, 14, 16, the acquisition and processing of the motion measurement data. For example, upon triggering the start of the exercise via a user input through the user interface, the exercise controller may control the motion sensors to start measuring the user's motion and to provide the motion measurement data. Upon acquiring a determined amount of motion measurement data, the controller 108 may activate the effectiveness estimation circuitry 106 to compute the effectiveness according to any one of the above-described embodiments. In embodiments where the effectiveness is computed after the exercise, the exercise controller 108 may, upon detecting the end of the exercise from the user input or otherwise, configure the effectiveness estimation circuitry to compute overall effectiveness of the motion conducted during the physical exercise from an aggregate of the computed motion effectiveness values or from all the motion measurement data acquired during the physical exercise. Either circuitry 106, 108 may carry out the monitoring in the embodiment of Figure 6. And as described above, the effectiveness may be output via the user interface 124, via the communication circuitry 102, and/or via a memory interface to a measurement database 119.

The memory 110 may store a computer program product 118 defining the motion effectiveness estimation algorithm the processor executes upon reading the computer program. The memory may further store a user profile 114 of the user 20 storing personal characteristics of the user 20, e.g. age, weight, etc. As described above, the weight may be used in the running power correction, for example. The memory may further store the measurement database 119 comprising the measured motion measurement data and the motion effectiveness parameters computed during and/or after the exercise.

As used in this application, the term 'circuitry' refers to all of the following: (a) hardware-only circuit implementations, such as implementations in only analog and/or digital circuitry, and (b) combinations of circuits and soft-ware (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network de-vice, or another network device.

In an embodiment, at least some of the processes described in connection with Figures 3 to 7 may be carried out by an apparatus comprising corresponding means for carrying out at least some of the described processes. Some example means for carrying out the processes may include at least one of the following: detector, processor (including dual-core and multiple-core processors), digital signal processor, controller, receiver, transmitter, encoder, decoder, memory, RAM, ROM, software, firmware, display, user interface, display circuitry, user interface circuitry, user interface software, display software, circuit, and circuitry. In an embodiment, the at least one processor 100, the memory 110, and the computer program code 118 form processing means or comprises one or more computer program code portions for carrying out one or more operations according to any one of the embodiments of Figures 3 to 8 or operations thereof.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chipset (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

Embodiments as described may also be carried out in the form of a computer process defined by a computer program or portions thereof. Embodiments of the methods described in connection with Figures 3 to 7 may be carried out by executing at least one portion of a computer program comprising corresponding instructions. The computer program may be in source code form, object code form, or in some intermediate form, and it may be stored in some sort of carrier, which may be any entity or device capable of carrying the pro-gram. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. The computer pro-gram medium may be a non-transitory medium. Coding of software for carrying out the embodiments as shown and described is well within the scope of a per-son of ordinary skill in the art.

It will be obvious to a person skilled in the art that, as the technology advances, the inventive concept can be implemented in various ways. The invention and its embodiments are not limited to the examples described above but may vary within the scope of the claims.

## Claims

1.   A computer-implemented method for estimating effectiveness of periodic motion, comprising:

measuring, by using at least one motion sensor, periodic motion of a user and thus acquiring motion measurement data during a time interval of a physical exercise;
transforming the motion measurement data into frequency-domain samples;
extracting, amongst the frequency-domain samples by using peak detection, a first subset of frequency-domain samples representing periodic motion;
computing a metric indicating a ratio between energy on the first subset of frequency domain samples and energy on other frequency domain samples; and
mapping the computed ratio to an effectiveness parameter by using a determined mapping rule and outputting the effectiveness parameter via an interface.

2.   The computer-implemented method of claim 1, wherein the first subset comprises a plurality of frequency-domain samples at harmonic signal components of a base frequency associated with the highest peak in the frequency-domain samples, and the mapping rule maps a ratio indicating more energy in the first subset of frequency-domain samples to a greater effectiveness parameter.

3.   The computer-implemented method of claim 2, wherein the first subset further comprises a frequency sample associated with the highest peak in the frequency-domain samples.

4.   The computer-implemented method of claim 2, wherein the metric is total harmonic distortion, and wherein the mapping rule maps a greater total harmonic distortion to a greater effectiveness parameter.

5.   The computer-implemented method of claim 1, further comprising:

measuring, by using the at least one motion sensor, further periodic motion of the user and thus acquiring further motion measurement data during a further time interval of a physical exercise; transforming the further motion measurement data into further frequency-domain samples; extracting, amongst the further frequency-domain samples by using peak detection, a second subset of frequency-domain samples representing the periodic motion;

correlating the first subset with the second subset and using a result of the correlation as a further factor for the effectiveness parameter such that a greater correlation is mapped to a greater effectiveness parameter while lower correlation is mapped to a lower effectiveness parameter.

6. The computer-implemented method of any preceding claim, wherein the time interval spans over the whole physical exercise.

7. The computer-implemented method of any preceding claim, wherein said method is performed by a wearable training computer repeatedly over multiple time intervals during the physical exercise to acquire multiple effectiveness parameters for the physical exercise, and wherein the effectiveness parameters are output via a display unit of the wearable training computer.

8. The computer-implemented method of claim 6, further comprising outputting, after an end of the exercise, a display view indicating the computed effectiveness parameters along a route of the physical exercise.

9. The computer-implemented method of any preceding claim, wherein the effectiveness parameter is a running effectiveness parameter used as a correction factor for a measured running power parameter and computing mechanical load of the exercise on the basis of the corrected running power parameter.

10. The computer-implemented method of any preceding claim, wherein the periodic motion is running and the effectiveness parameter is a running effectiveness parameter, the method further comprising monitoring for changes in the running effectiveness parameter and, upon detecting a degradation in the running effectiveness parameter, performing the following:

acquiring data indicating a terrain type associated with the measured periodic motion and determining the terrain type;
if the terrain type is determined to be non-flat, ignoring the change in the running effectiveness parameter; and
if the terrain type is determined to be flat, outputting an indication to the user of the degradation in the running effectiveness.

11. The computer-implemented method of any preceding claim, wherein the at least one motion sensor comprises a plurality of motion sensors configured to measure the motion in three spatial dimensions X, Y, Z and to generate the motion measurement data, where X represents motion towards a general direction the user is moving, Y represents lateral motion of the user towards a direction that is perpendicular to the X dimension, and Z represents motion to a direction that is perpendicular to both X and Y directions, the method further comprising computing, on the basis of the motion measurement data, a motion vector for each spatial dimension X, Y, Z and determining the effectiveness further on the basis of the motion vectors such that the running effectiveness is directly proportional to the magnitude of the vector in X dimension and inversely proportional to the magnitude of the vectors in the Y and Z dimensions.

12. A computer program product readable by a computer and comprising program instructions which, when executed by the computer, cause the computer to carry out a computer process implementing all the steps of the method according to any preceding claim 1 to 11.

13. A computer system for estimating effectiveness of periodic motion, comprising:

means for receiving, from at least one motion sensor, motion measurement data representing periodic motion of a user during a time interval of a physical exercise;
means for transforming the motion measurement data into frequency-domain samples;
means for extracting, amongst the frequency-domain samples by using peak detection, a first subset of frequency-domain samples representing periodic motion;
means for computing a metric indicating a ratio between energy on the first subset of frequency domain samples and energy on other frequency domain samples; and
means for mapping the computed ratio to an effectiveness parameter by using a determined mapping rule and outputting the effectiveness parameter via an interface.

14. The computer system of claim 13, further comprising means for performing all the steps of the method of any one of claims 2 to 11.

12: TRAINING COMPUTER
(MOTION SENSOR(S))

Fig 1

START

12/14/15/16

200: ACQUIRE PERIODIC MOTION
MEASUREMENT DATA

MOTION
MEASUREMENT DATA

202: FFT

204: EXTRACT PEAKS

206: COMPUTE METRIC INDICATING RATIO BETWEEN ENERGY ON PEAKS
COMPONENT(S) AND ENERGY ON OTHER COMPONENT(S)

208: MAP RATIO TO EFFECTIVENESS AND
OUTPUT EFFECTIVENESS

DATABASE

END

Fig 2

MAGNITUDE

PEAKS (BASE FREQUENCY
& HARMONICS)

FREQUENCY

Fig 3A

EFFICIENT RUNNING

Fig 3B

INEFFICIENT RUNNING

Fig 3C

400

402

80%

60%

90%

80%

Fig 4

START

200-208

500: COMPUTE RUNNING POWER
(SPEED, LOCATION/ALTITUDE GRADIENT)

502:?

YES

504: COMPUTE CORRECTION VALUE FOR RUNNING POWER &
OUTPUT CORRECTED RUNNING POWER METRIC

END

Fig 5

START

200-208

602: MONITOR CHANGE IN EFFECTIVENESS

604: >THRESHOLD?

NO

YES

606: ACQUIRE TERRAIN TYPE (GNSS)

608: FLAT?

610: OUTPUT NOTIFICATION OF DEGRADING EFFECTIVENESS

Fig 6

START

200

700: EXTRACT X, Y, Z COMPONENTS

702: COMPUTE MOTION VECTOR TO X, Y, Z DIMENSIONS

208

704: COMPUTE EFFECTIVENESS
- HIGH ENERGY IN PERIODIC COMPONENTS & HIGH X COMPONENT→ HIGH EFFECTIVENESS
- HIGH ENERGY IN PERIODIC COMPONENTS & LOW X COMPONENT→ LOW EFFECTIVENESS (SLOPE?)
- LOW ENERGY IN PERIODIC COMPONENTS & HIGH X COMPONENT→ LOW EFFECTIVENESS (TECHNIQUE?)
- LOW ENERGY IN PERIODIC COMPONENTS & LOW X COMPONENT→ VERY LOW EFFECTIVENESS

END

Fig 7

Fig 8

Europäisches
Patentamt
European
Patent Office
Office européen
des brevets

# EUROPEAN SEARCH REPORT

Application Number

EP 21 18 4993

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | JP 2020 151470 A (KOBE GAKUIN)<br>24 September 2020 (2020-09-24)<br>* paragraphs [0001], [0019], [0044] -<br>[0049], [0058], [0068], [0070]; figures<br>1-6 * | 1-8,<br>11-14<br>9,10 | INV.<br>A61B5/11<br>A61B5/00<br>A63B69/00 |
| X<br><br><br><br><br><br><br><br><br><br>A | PASCIUTO ILARIA ET AL: "Overcoming the limitations of the Harmonic Ratio for the reliable assessment of gait symmetry",<br>JOURNAL OF BIOMECHANICS,<br>vol. 53, 1 February 2017 (2017-02-01),<br>pages 84-89, XP055865934,<br>US<br>ISSN: 0021-9290, DOI:<br>10.1016/j.jbiomech.2017.01.005<br>* Abstract, sections 2.1-2.3 , equations 1-2 * | 1-8,<br>11-14<br><br><br><br><br><br><br><br><br>9,10 | |
| X<br><br><br><br><br><br><br><br><br><br><br>A | ALICIA MART?NEZ-RAM?REZ ET AL: "Frailty assessment based on trunk kinematic parameters during walking",<br>JOURNAL OF NEUROENGINEERING AND REHABILITATION, BIOMED CENTRAL , LONDON, GB,<br>vol. 12, no. 1, 24 May 2015 (2015-05-24),<br>page 48, XP021223152,<br>ISSN: 1743-0003, DOI:<br>10.1186/S12984-015-0040-6<br>* Abstract, p.2-3 section "testing procedures, walking test, instrumentation, acceleration parameters;<br>page 1 - page 3 * | 1-8,<br>11-14<br><br><br><br><br><br><br><br><br><br>9,10 | TECHNICAL FIELDS SEARCHED (IPC)<br><br>A61B<br>A63B |
| A | US 2016/192866 A1 (NORSTROM CHRISTER [SE] ET AL) 7 July 2016 (2016-07-07)<br>* paragraphs [0036], [0040] - [0041]; figure 2a * | 1-14 | |

-/--

The present search report has been drawn up for all claims

| Place of search | Date of completion of the search | Examiner |
|---|---|---|
| The Hague | 1 December 2021 | Hirsch, Arthur |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

**EUROPEAN SEARCH REPORT**

Application Number

EP 21 18 4993

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/156215 A1 (EASTMAN KYLER [US] ET AL) 5 June 2014 (2014-06-05) * paragraphs [0029], [0034], [0039], [0049], [0096]; figures 1-10 * ----- | 1-14 | |
| A | EP 1 754 521 A1 (SUISSE ELECTRONIQUE MICROTECH [CH]) 21 February 2007 (2007-02-21) * Equations 3-5; paragraphs [0011], [0017], [0022], [0025] * ----- | 1-14 | |

TECHNICAL FIELDS
SEARCHED      (IPC)

The present search report has been drawn up for all claims

| Place of search | Date of completion of the search | Examiner |
|---|---|---|
| The Hague | 1 December 2021 | Hirsch, Arthur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 4993

This annex lists the patent family members relating to the patent documents cited in  the above-mentioned European search report.
The members are as contained in the European Patent Office EDP file on
The European Patent Office is in no way liable for these particulars which are merely given for the purpose of information.

01-12-2021

| Patent document cited in search report | | Publication date | Patent family member(s) | | Publication date |
|---|---|---|---|---|---|
| JP 2020151470 | A | 24-09-2020 | NONE | | |
| US 2016192866 | A1 | 07-07-2016 | SE | 1350894 A1 | 19-01-2015 |
| | | | US | 2016192866 A1 | 07-07-2016 |
| | | | WO | 2015009228 A1 | 22-01-2015 |
| US 2014156215 | A1 | 05-06-2014 | US | 2014156215 A1 | 05-06-2014 |
| | | | WO | 2014089238 A1 | 12-06-2014 |
| EP 1754521 | A1 | 21-02-2007 | AT | 433341 T | 15-06-2009 |
| | | | EP | 1754521 A1 | 21-02-2007 |

EPO FORM P0459

For more details about this annex : see Official Journal of the European Patent Office, No. 12/82